# EUROPÄISCHE PATENTSCHRIFT

(11) **EP 0 775 698 B1**
(45) Veröffentlichungstag und Bekanntmachung des Hinweises auf die Patenterteilung: **24.09.2003**
(21) Anmeldenummer: 96810796.1
(22) Anmeldetag: 14.11.1996
(51) Int. Cl.: C07D 251/24, A61K 7/42, C07D 251/22, C07D 403/04, C07F 7/08

(54) **Bis-Resorcinyl-Triazine als UV-Absorber**
Bis-resorcinol-triazines as UV absorber triazine
Bis-résorcinol-triazines comme UV-absorbants

(30) Priorität: 23.11.1995 DE 19543730
(43) Veröffentlichungstag der Anmeldung: 28.05.1997
(73) Patentinhaber: Ciba Specialty Chemicals Holding Inc., 4057 Basel (CH)
(72) Erfinder: Hüglin, Dietmar, 79104 Freiburg (DE); Borsos, Elek, 4127 Birsfelden (CH); Luther, Helmut, 79639 Grenzach-Wyhlen (DE); Herzog, Bernd, 79639 Grenzach-Wyhlen (DE); Bachmann, Frank, 79106 Freiburg (DE)

(56) Entgegenhaltungen:
- EP-A- 0 165 608
- EP-A- 0 531 258
- WO-A-94/18278
- DE-A- 4 340 725
- GB-A- 2 286 774
- US-A- 4 826 978

## Beschreibung

Die vorliegende Erfindung betrifft neue Bis-Resorcinyl-Triazine, Verfahren zur Herstellung dieser Verbindungen sowie die Verwendung von ausgewählten Bis-Resorcinyl-Triazinen für kosmetische Mittel.

In der US-A-3,268,474 werden Polymerzusammensetzungen offenbart, die als UV-Filter s-Triazinverbindungen enthalten.

Die US-A-3,244,708 und US-A-3,242,175 offenbaren Hydroxyphenyl-1,3,5-s-Trtiazine, die u.a. als UV-Filter in kosmetischenZusammensetzungen eingesetzt werden können.

Die DE-A-4340725 offenbart Triazinderivate, die in photografischen Materialien, Tinten bzw. Aufzeichnungsmaterialien für Tintenstrahldruck und Lacken verwendet werden.

Die EP-A-0,531,258 offenbart photographisches Material, das s-Triazinverbindungen als UV-Absorber enthält.

Die WO 94/18278 opffenbart Tris-aryl-s-triazinverbindungen, die in Polymerzusammensetzungen Verwendung finden.

Die GB-A-2,288,774 offenbart Sonnenschutzmittel, die als UV-Absorber organische Verbindungen, wie z.B. Triazinverbindungen, in mikronisierter Form enthalten.

Die neuen Bis-Resorcinyl-Triazine entsprechen der Formel 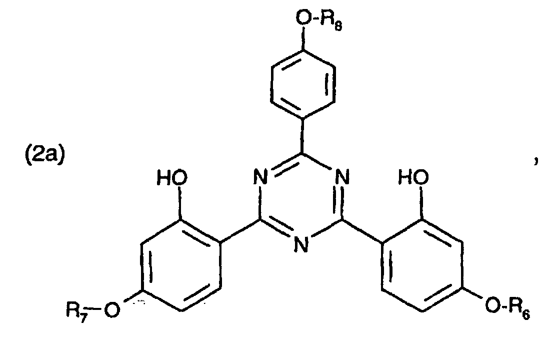 oder 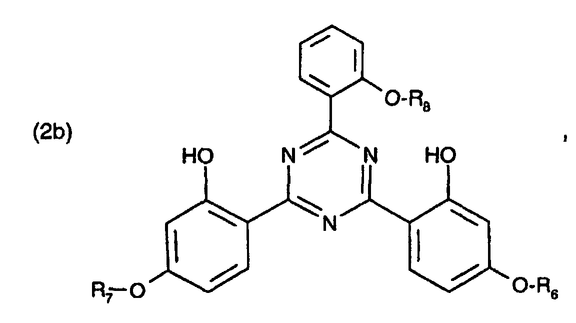 worin
R₆ und R₇, C₃-C₁₈-Alkyl;
R₈ C₁-C₁₀-Alkyl
bedeuten.

C₁-C₅-Alkyl, C₁-C₈-Alkyl, C₁-C₁₀-Alkyl, bzw C₃-C₁₈-Alkyl sind geradkettige oder verzweigte Alkylreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl, Pentadecyl, Hexadecyl, Heptadecyl oder Octadecyl.

C₂-C₁₈-Alkenyl bedeutet z.B. Allyl, Methallyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Dodec-2-enyl oder n-Octadec-4-enyl.

Als Beispiele für Verbindungen der Formel (1) seien genannt:
2-(4'-Methoxyphenyl)-4,6-bis(2'-hydroxy-4'-n-octyloxyphenyl)-1,3,5-triazin.

Die neuen Bis-Resorcinyl-Triazine lassen sich auf verschiedene Art und Weise herstellen. Beispielsweise lassen sich die Verbindungen der Formel (2a) oder (2b), in einer dreistufigen Reaktion, ausgehend von Cyanurchlorid, herstellen. Man setzt dabei die entsprechende Phenylmagnesiumbromidverbindung in einer Grignardreaktion mit Cyanurchlorid zur Dichlortriazinverbindung der Formel 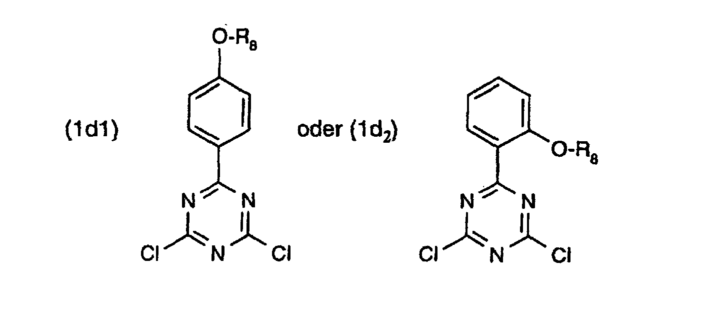 um. Verfahren zur Herstellung dieser Zwischenstufe sind bekannt und z.B. in der EP-A-0,577,559 beschrieben. Anschliessend werden die beiden Resorcingruppen in allgemein bekannter Weise durch Friedel-Crafts-Acylierung von Resorcin in Gegenwart einer Lewis-Säure, insbesondere Aluminiumchlorid, eingeführt. In der dritten Stufe erfolgt die Veretherung der freien, p-ständigen Hydroxylgruppen, je nach Bedeutung der Reste R₆ und R₇, durch Alkylierung bzw. säurekatalysierte Addition von Glycidylethern. Ausführliche Angaben dazu können den Herstellungsbeispielen entnommen werden.

Die Dichlortriazin-Zwischenstufe der Formel (1d₁) bzw. (1d₂) ist auch ohne Einsatz von Grignard-Reagenzien durch Ringschlussreaktion zugänglich. Dazu wird das entsprechend substituierte Benzonitril mit Dicyandiamid zum 6-Aryl-1,3,5-triazin-2,4-dion umgesetzt, welches mit Thionylchlorid in das Chlorderivat der Formel (1d₁) bzw. (1d₂) übergeführt wird. Alternativ dazu ist die Verbindung der Formel (1d) auch durch Reaktion der entsprechend substituierten N,N-Dimethyl-carbonsäureamide mit Phosphoroxychlorid und N-Cyan-chlorformamidin zugänglich. Diese Reaktionen sind bereits bekannt und z.B. in Dyes and Pigments 7, 419-443 (1986) beschrieben.

Verbindungen der Formel (2a) bzw. (2b), lassen sich weiterhin durch Umsetzung von phenylsubstituierten Benzoxazin-4-onen der Formel 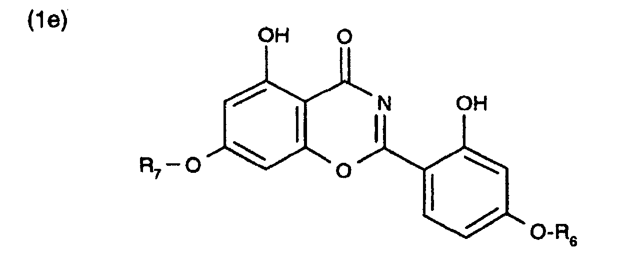 mit Benzamidinverbindungen der Formel 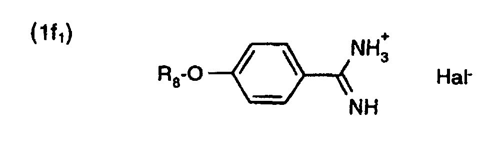 oder 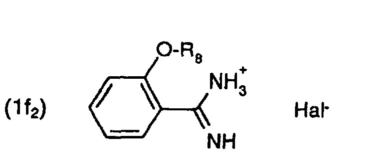 erhalten, wobei R₁, R₂ und R₃ die angegebene Bedeutung haben. Die Herstellung solcher Benzoxazinon-Zwischenstufen und die Umsetzung mit Amidinen sind in Helv.Chim.Acta 55, 1566-1595 (1972) beschrieben.

Weiterhin können die erfindungsgemässen Verbindungen der Formel (2a) oder (2b) durch Dehydrierung einer Dihydrotriazinverbindung der Formel 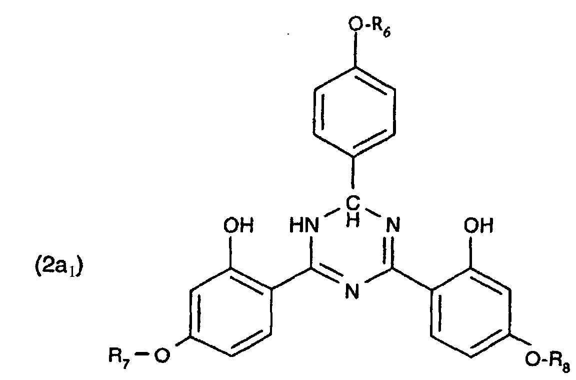 hergestellt werden.
R₆, R₇ und A₁ haben dabei die in Formel (2a) und (2b) angegebene Bedeutung.

Als Dehydrierungsmittel wird in der Regel Chloranil eingesetzt. Die Dehydrierung von Dihydrotriazinverbindungen zu 1,3,5-Triazinen mit Hilfe von Chloranil ist z.B. aus der Khim. Geteritsikl. Soedin. (2), S. 350-353 (1969) bekannt.

Die erfindungsgemässen Verbindungen der Formel (2a) und (2b) sowie weitere, aus dem Stand der Technik bekannte ausgewählte Bis-Resorcinyl-Triazinverbindungen eignen sich insbesondere als UV-Filter, d.h. zum Schützen von ultraviolett empfindlichen organischen Materialien, insbesondere der Haut und Haare von Menschen und Tieren vor der schädigenden Einwirkung von UV-Strahlung. Diese Verbindungen eignen sich daher als Lichtschutzmittel in kosmetischen, pharmazeutischen und veterinärmedizinischen Präparaten. Diese Verbindungen können sowohl gelöst als auch im mikronisierten Zustand verwendet werden.

Einen weiteren Erfindungsgegenstand bildet daher ein kosmetisches Präparat, enthaltend mindestens eine Verbindung der Formel 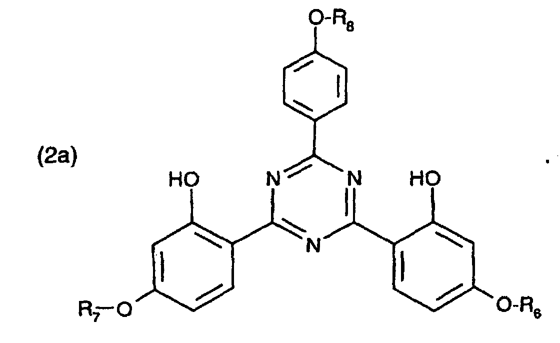 oder 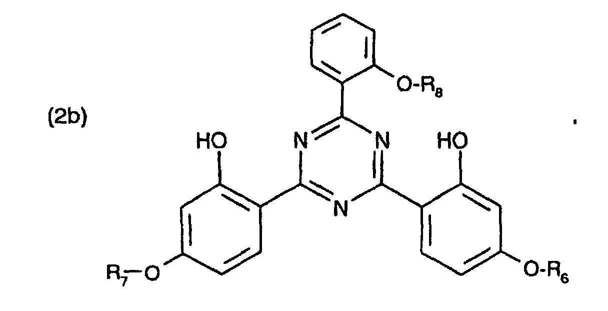 worin
R₆ und R₇, C₃-C₁₈-Alkyl;
R₈ C₁-C₁₀-Alkyl
bedeuten;
sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Die kosmetischen Zusammensetzungen können neben den erfindungsgemässen UV-Absorbem auch noch einen oder mehrere weitere UV-Schutzstoffe, wie z.B. Triazine, Oxanilide, Triazole oder Vinylgruppen enthaltende Amide oder Zimtsäureamide enthalten. Solche Schutzstoffe sind z.B. in der GB-A-2,286,774 beschrieben oder auch aus Cosmetics & Toiletries (107), 50ff (1992) bekannt.

Die erfindungsgemässen kosmetische Zusammensetzungen enthalten 0,1 bis 15, vorzugsweise 0,5 bis 10 Gew.-%, bezogen auf das Gesamtgewicht der Zusammensetzung, eines UV-Absorbers oder eines Gemisches aus UV-Absorbern und einen kosmetisch verträglichen Hilfsstoff.

Die Herstellung der kosmetischen Zusammensetzungen kann durch physikalisches Mischen des oder der UV-Absorber mit dem Hilfsstoff durch gewöhnliche Methoden, wie z.B. durch einfaches Zusammenrühren der Einzelkomponenten erfolgen.

Die erfindungsgemässen kosmetischen Zusammensetzungen können als Wasser-in-Öloder Öl-in-Wasser-Emulsion, als Öl-in-Alkohol-Lotion, als vesikulare Dispersion eines ionischen oder nichtionischen amphiphilen Lipids, als Gel, fester Stift oder als AerosolFormulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Für die erfindungsgemässen kosmetischen Zusammensetzungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die kosmetischen Zusammensetzungen können auch weitere Komponenten wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe enthalten.

Die erfindungsgemässen kosmetischen Formulierungen zeichnen sich durch exzellenten Schutz der menschlichen Haut gegen den schädigenden Einfluss von Sonnenlicht aus.

In den folgenden Beispielen beziehen sich die Prozentsätze auf das Gewicht. Die Mengen beziehen sich bei den eingesetzten Bis-Resorcinyl-Triazinverbindungen auf die Reinsubstanz.

### Herstellungsbeispiele der neuen Verbindungen:

### Beispiel 1: 2,4-Bis-{[4-(2-ethyl-hexyloxy)-2-hydroxy]-phenyl}-6-(4-methoxyphenyl)-1,3,5-triazin

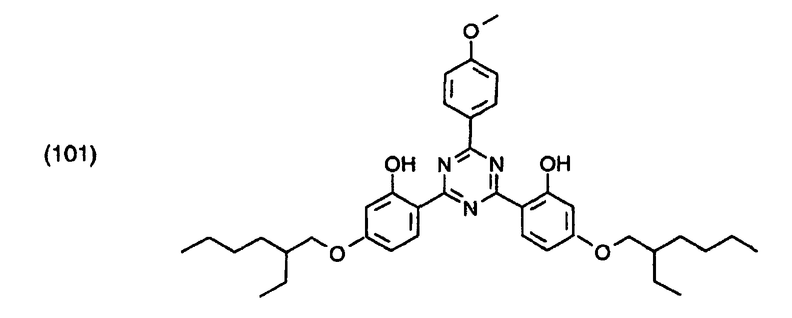

a) Grignard-Reaktion: In einem 500 ml Sulfierkolben mit Rührer, Tropftrichter, Kühler und Innenthermometer werden 7,3g (0,3 Mol) Magnesiumspäne und einige Körner lod in 60ml trockenem Tetrahydrofuran (THF) vorgelegt. Unter trockenem Schutzgas (Stickstoff) werden 56,1g (0,3 Mol) 4-Bromanisol, gelöst in 100ml THF bei 60°C langsam zugetropft. Nach dem vollständigen Auflösen der Mg-Späne wird 90 Minuten bei 60°C nachgerührt. Anschliessend tropft man die Grignard-Lösung bei 5°C innert 60 Minuten unter Stickstoff in eine Lösung von 55,3g (0,3 Mol) Cyanurchlorid in 150ml THF. Man dampft bei Raumtemperatur zur Trockene ein, lässt in 500ml verd. Salzsäure einlaufen und extrahiert mit 300ml Methylenchlorld. Zur Aufarbeitung wird das Lösungsmittel im Vakuum entfernt, der halbfeste Rückstand mit wenig Isopropanol verrieben und abgesaugt. Die Reinigung erfolgt durch Umkristallisation aus Toluol / Hexan (1/1). Man erhält 62,8g (82% d.Th.) 6-(4-Methoxyphenyl)-2,4-dichlor-1,3,5-triazin (Smp.: 131-134°C).
b) Friedel-Crafts-Acylierung: In einem 750ml Sulfierkolben mit Rührer, Tropftrichter, Kühler und Innenthermometer werden 43,5g (0,17 Mol) 6-(4-Methoxyphenyl)-2,4-dichlor-1,3,5-tria zin und 37,5g (0,34 Mol) Resorcin in einer Mischung aus 150ml Xylol (Isomerengemisch) und 150ml Sulfolan vorgelegt. Bei 60-65°C trägt man langsam 52,1g (0,39 Mol) Aluminiumchlorid ein und rührt, bis die HCI-Entwicklung beendet ist (ca. 4 Stunden). Man lässt die warme Reaktionslösung unter Rühren in 750ml Methanol/350ml verd. Salzsäure einlaufen, saugt ab und wäscht mit Wasser neutral. Die Trocknung erfolgt bei 100°C im Vakuum. Man erhält 61,1g (89,1% d.Th.) 2,4-Bis-(2,4-dihydroxy-phenyl)-6-(4-methoxyphenyl)-1,3,5-triazin als gelbes Pulver.
c) Alkylierung: In einem 500ml Sulfierkolben mit Rührer, Tropftrichter, Kühler und Innenthermometer werden 20,2g (0,05 Mol) 2,4-Bis-(2,4-dihydroxy-phenyl)-6-(4-methoxyphenyl)-1,3,5-triazin zusammen mit 200ml Methylcellosolve (Merck®) und 8,8g (0,11 Mol) 50%-ige Natronlauge vorgelegt. Man rührt 30 Minuten lang bei 80°C und tropft bei derselben Temperatur 23,2g (0,12 Mol) 3-Brommethyl-heptan, gelöst in 25ml Methylcellosolve, langsam zu. Die Alkylierung lässt sich dünnschichtchromatographisch verfolgen. Nach 8 Stunden Reaktion bei 112-114°C kann das Edukt nicht mehr nachgewiesen werden. Man dampft zur Trockne ein, nimmt in 100ml Toluol/Hexan (7 vol./3 vol.) auf und filtriert vom Ungelösten ab. Zur Reinigung wird über Kieselgel (Säule: ⌀=5cm, l=60cm) chromatographiert. Man erhält die Verbindung der Formel (101) als zähes, leicht gelbes Harz, welches nach einigen Wochen auskristallisiert. Ausbeute: 24,6g (78.4% d.Th.). Die Kristallisation kann durch Zugabe von Impfkristallen beschleunigt werden.

Eigenschaften:
schwach gelbe Kristalle, Smp.: 83-85°C
UV-Spektrum: λₘₐₓ = 343nm, λₘₐₓ = 47 000 M⁻¹cm⁻¹ (Ethanol)
¹H-NMR-Spektrum: δ [ppm, CDCl₃] = 0.8-1.0 (m, 12H, -CH₃), 1.2-1.8 (m, 18H, -CH2- und
-CH-), 3.8-3.9 (d, s, 7H, -O-CH₂- und -O-CH₃), 6.4-8.6 (10H, Aromaten), 13.2-13.6 (s, 2H, -OH)

### Beispiel 2: Man verfährt wie in Beispiel 1c) beschrieben, mit dem Unterschied, dass man für die Alkylierung anstelle von 3-Brommethylheptan 17,8 g 3-Chloromethylheptan, gelöst in 25ml DMF verwendet.

### Applikationsbeispiele

### Anwendungsbeispiele für kosmetischen Lichtschutz

Die Lichtschutzfaktoren wurden bestimmt gemäss der Methode von Diffey und Robson, J. Soc. Cosmet. Chem. 40, 127 - 133 (1989) mit einem SPF-Analysator (Optometrix, SPF 290).

Zur Bestimmung der Photostabilitäten werden die Filtersubstanzen in Ethanol gelöst (c = 1·10⁻⁵ - 5·10⁻⁵ M) und in einer Quartz-Küvette unter Rühren mit einer Metallhalogenidlampe (Macam) bestrahlt (I_{UVB}= 0,4 - 8,0 mW/cm²). Zur Umrechnung auf das Sonnenspektrum (CIE D65-Normtageslicht, normiert auf I_{UVB}= 0,127 mW/cm²) wird das Integral über die Produkte der wellenlängenaufgelösten Lampenintensität mit den entsprechenden Absorptionswerten des jeweiligen UV-Absorbers zwischen 290 und 400 nm berechnet und durch das Integral über die Produkte der D65-Lichtintensitäten mit den entsprechenden Absorptionswerten des jeweiligen UV-Absorbers im Bereich zwischen 290 und 400 nm dividiert. Mit diesem Faktor wird die Halbwertszeit für den Abbau unter Bestrahlung mit der Metallhalo genidlampe multipliziert, um die entsprechende Halbwertszeit unter Sonneneinstrahlung zu erhalten. Die Halbwertszeit für den Photoabbau unter Lampeneinstrahlung wird über UVspektroskopische Messung der Extinktion bei der Wellenlänge der Maximalabsorption und anschliessenden Exponentialfit bestimmt. Mit dem beschriebenen Verfahren erhält man also die Halbwertszeiten für den Photoabbau im D65-Licht.

### Beispiel 11: o/w-Emulsion mit der Verbindungen der Formel (101)

| (A): | |
|---|---|
| Triazin-UV-Absorber | 3 g |
| Sesamöl | 10 g |
| Glycerylstearat | 4 g |
| Stearinsäure | 1 g |
| Cetylalkohol | 0,5 g |
| Polysorbat 20 | 0,2 g |
| | |

| (B): | |
|---|---|
| Propylenglykol | 4 g |
| Propylparaben | 0,05 g |
| Methylparaben | 0,15 |
| Triethanolamin | 0,1 g |
| Carbomer 934 | 0,1 g |
| Wasser | ad 100 ml |

### Herstellung der Emulsion

### Phase (A):

Zunächst wird der UV-Absorber in Sesamöl gelöst. Die anderen Komponenten von (A) werden dazugegeben und zusammengeschmolzen.

### Phase (B):

Propylparaben und Methylparaben werden im Propylenglykol gelöst. Danach werden 60ml Wasser zugegeben, auf 70°C erhitzt und Carbomer 934 darin emulgiert.
Emulsion:
(A) wird langsam unter starkem mechanischem Energieeintrag zu B gegeben. Das Volumen wird durch Zugabe von Wasser auf 100 ml eingestellt.

Die ermittelten Sonnenschutzfaktoren und Photostabilitäten sind aus Tabelle 1 zu entnehmen.

**Tabelle 1:**

| | Konzentration | Sonnenschutzfaktor *) | Photostabilität*) [h] |
|---|---|---|---|
| Verbindung der Formel (101) | 3% | 9,1 | 1500 |

| | | | |
|---|---|---|---|
| *) nach Diffey und Robson **) als Halbwertszeit des Photoabbaus im D65-Licht in ethanolischer Lösung | | | |

Der Sonnenschutzfaktor kann mit der UV-Absorber-Konzentration variiert werden.

Die Ergebnisse zeigen, dass die Wirksubstanzen eine hohe Photostabilität aufweisen und schon mit niedriger Konzentration ein guter Sonnenschutzfaktor erzielt werden kann.

## Patentansprüche

1. Bis-Resorcinyl-Triazine der Formel 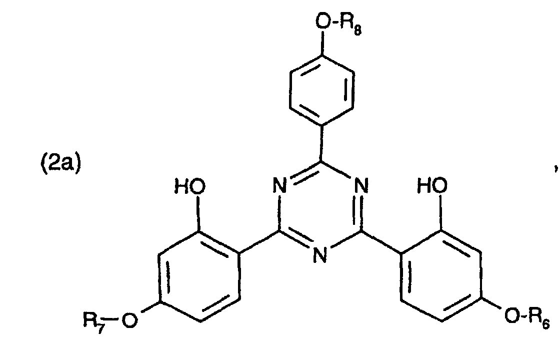 oder 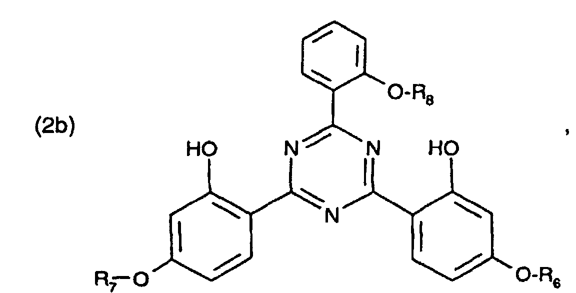 worin
R₆ und R₇, C₃-C₁₈-Alkyl;
R₈ C₁-C₁₀-Alkyl
bedeuten.

2. Verfahren zur Herstellung der Bis-Resorcinyl-Triazine der Formel (2a) oder (2b), durch Umsetzung der entsprechenden Phenylmagnesiumbromidverbindung in einer Grignardreaktion mit Cyanurchlorid zur Dichlortriazinverbindung der Formel 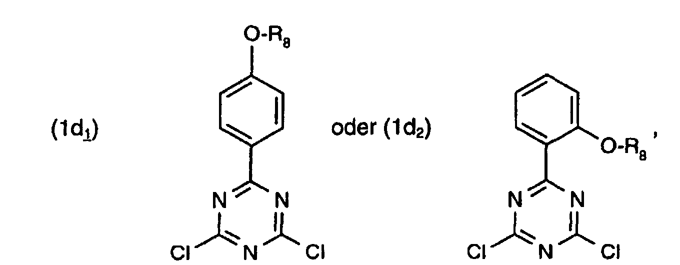 Einführung der Resorcingruppen durch Friedel-Crafts-Acylierung von Resorcin in Gegenwart einer Lewis-Säure, insbesondere Aluminiumchlorid, und Veretherung der freien, p-ständigen Hydroxylgruppen, je nach Bedeutung der Reste R₆ und R₇, durch Alkylierung bzw. säurekatalysierte Addition von Glycidylethern.

3. Verfahren zur Herstellung der Bis-Resorcinyl-Triazine nach Anspruch 1 durch Umsetzung von phenylsubstituierten Benzoxazin-4-onen der Formel 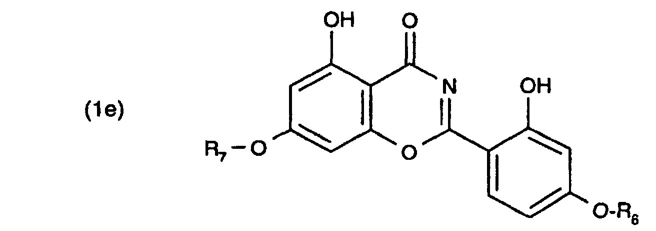 mit Benzamidinverbindungen der Formel 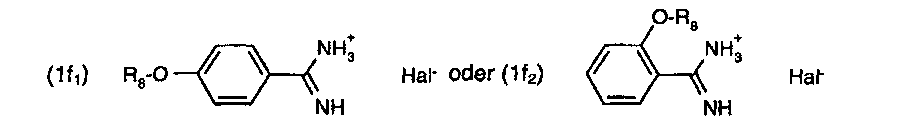 wobei
R₃, R₇ und R₈ die in Formel (1) angegebene Bedeutung haben.

4. Verfahren zur Herstellung der Verbindungen der Formel (1) durch Dehydrierung einer Dihydrotriazinverbindung der Formel 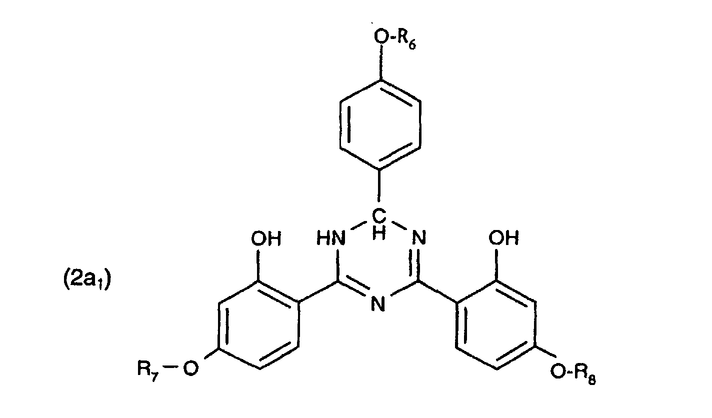 oder 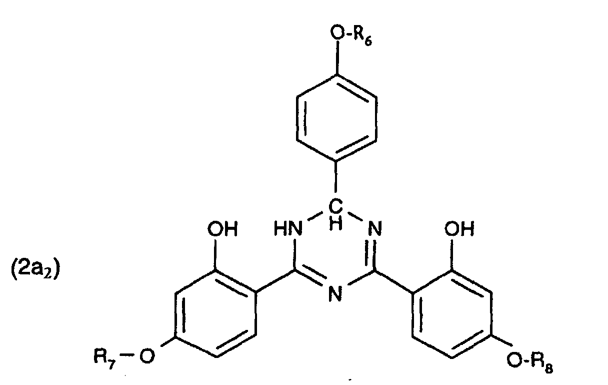 worin
R₁₆, R₂₇ und A₁ und R₆ die in Formel (1) angegebene Bedeutung haben.

5. Nichttherapeutische Verwendung der Bis-Resorcinyl-Triazine der Formel (2a) und (2b) nach Anspruch 1
zum Schützen von menschlichen und tierischen Haaren und Haut vor der schädigenden Einwirkung von UV-Strahlung.

6. Kosmetisches Präparat, enthaltend mindestens eine oder mehrere Verbindungen der Formel (2a) und (2b) nach Anspruch 5 mit kosmetisch verträglichen Träger- oder Hilfsstoffen.

7. Präparat nach Anspruch 6, **dadurch gekennzeichnet, dass** es weitere UV-Schutzstoffe enthält.

8. Präparat nach Anspruch 6 oder 7, **dadurch gekennzeichnet, dass** es als weitere UV-Schutzstoffe Triazine, Oxanilide, Triazole, Vinylgruppen enthaltende Amide oder Zimtsäureamide enthält.

## Claims

1. A bis(resorcinyl)triazine of the formula 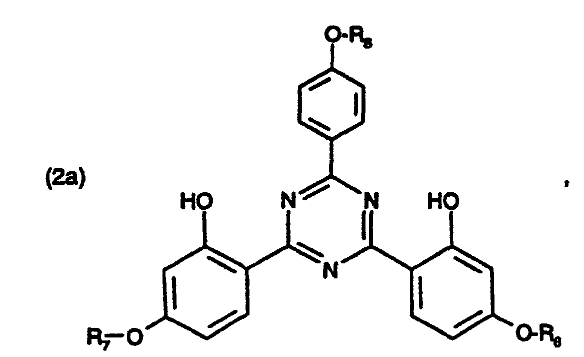 or 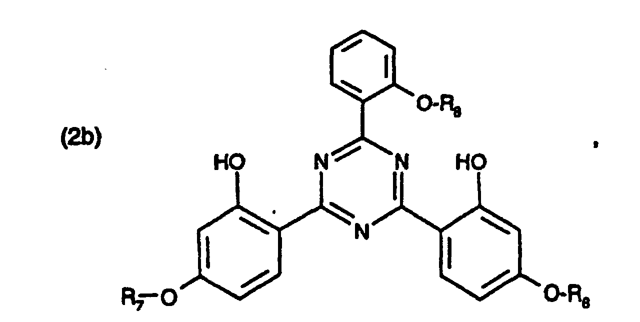 in which
R₆ and R₇ are C₃-C₁₈alkyl;
R₈ is C₁-C₁₀alkyl.

2. A process for the preparation of the bis(resorcinyl)triazines of the formula (2a) or (2b) by reaction of the corresponding phenylmagnesium bromide compound in a Grignard reaction with cyanuric chloride to give the dichlorotriazine compound of the formula 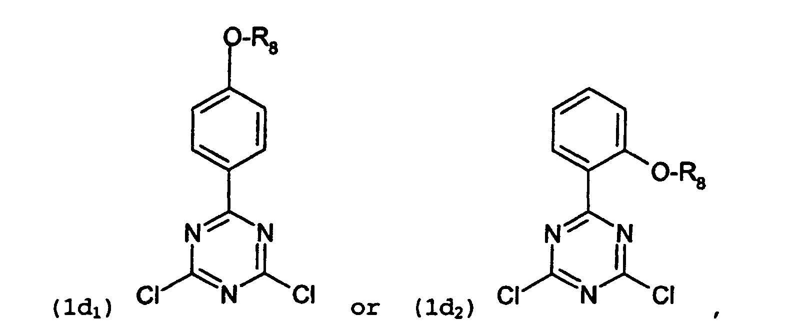 introduction of the resorcinol groups by Friedel-Crafts acylation of resorcinol in the presence of a Lewis acid, in particular aluminium chloride, and etherification of the free p-hydroxyl groups, depending on the meaning of the radicals R₆ and R₇, by alkylation or acid-catalysed addition reaction of glycidyl ethers.

3. A process for the preparation of the bis(resorcinyl)triazines according to claim 1 by reaction of phenyl-substituted benzoxazin-4-ones of the formula 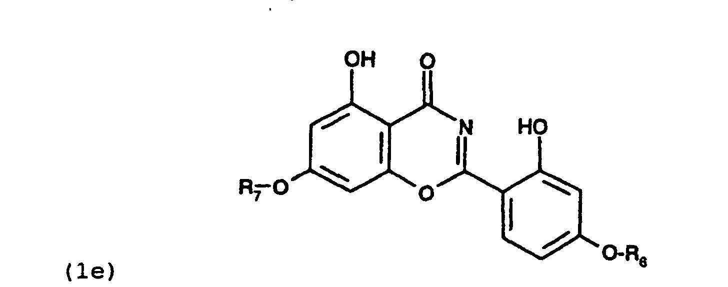 with benzamidine compounds of the formula 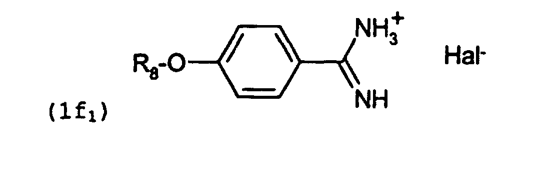 or 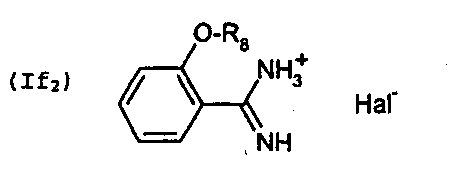 where
R₆, R₇ and R₈ are as defined for formula (2a) or (2b).

4. A process for the preparation of the compounds of the formula (2a) or (2b) by dehydrogenation of a dihydrotriazine compound of the formula 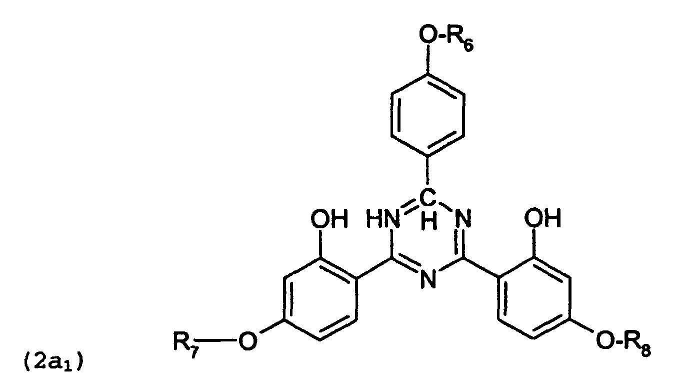 or 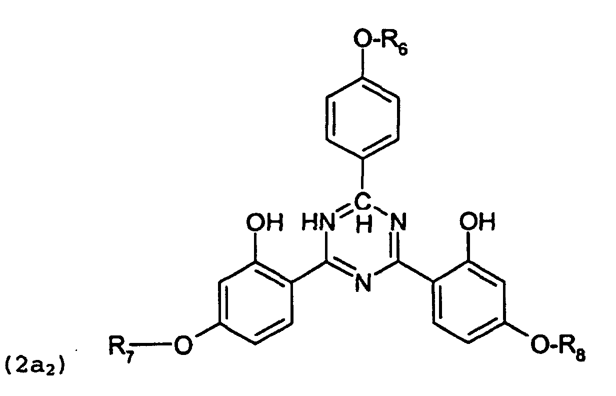 in which
R₆, R₇ and R₈ are as defined for formula (2a) or (2b).

5. The non-therapeutic use of the bis(resorcinyl)-triazines of the formula (2a) and (2b) according to claim 1 for protecting human and animal hair and skin from the harmful effects of UV radiation.

6. A cosmetic preparation, comprising at least one or more compounds of the formula (2a) and (2b) according to claim 5 with cosmetically tolerable carriers or auxiliaries.

7. A preparation according to claim 6, which comprises further UV-protective substances.

8. A preparation according to claim 6 or 7, which, as further UV-protective substances, comprises triazines, oxanilides, triazoles, amides containing vinyl groups, or cinnamamides.

## Revendications

1. Bis-résorcinyl-triazines de formule 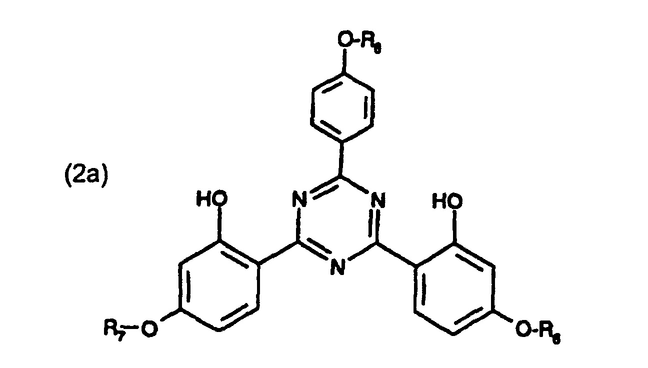 ou 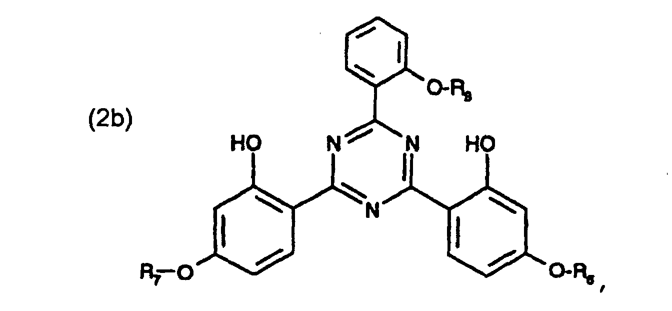 dans lesquelles
R₆ et R₇ représentent un groupe alkyle en C₃ à C₁₈ ;
R₈ représente un groupe alkyle en C₁ à C₁₀.

2. Procédé pour la préparation des bis-résorcinyltriazines de formule (2a) ou (2b) par réaction du composé bromure de phénylmagnésium correspondant dans une réaction de Grignard avec le chlorure de cyanuryle pour obtenir le composé dichlorotriazine de formule 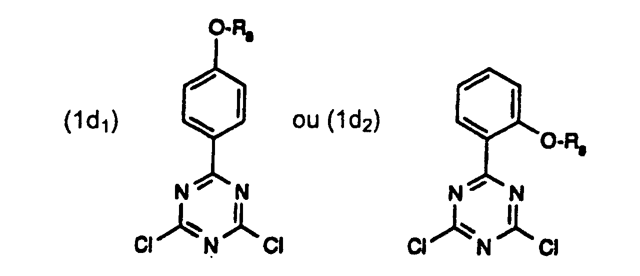 Introduction des groupes résorcine par acylation de Friedel-Crafts de résorcine en présence d'un acide de Lewis, en particulier le chlorure d'aluminium, et éthérification des groupes hydroxyle en position p, respectivement selon la signification des groupes R₆ et R₇, par alkylation ou addition catalysée en milieu acide de glycidyléthers.

3. Procédé pour la préparation de bis-résorcinyl-triazines selon la revendication 1 par réaction de benzoxazine-4-ones substituées par un phényle de formule 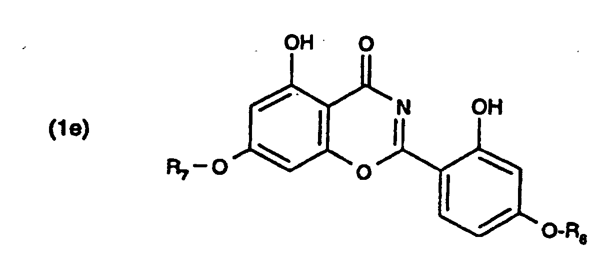 avec des composés benzamidine de formule 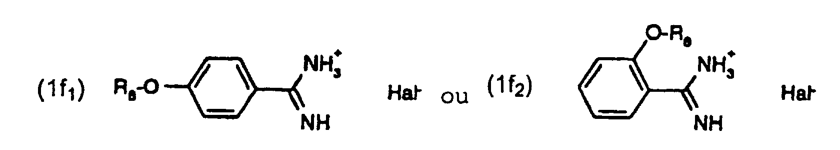 dans lesquelles
R₃, R₇ et R₈ ont les significations indiquées à la formule (1).

4. Procédé pour la préparation des composés de formule (1) par déshydrogénation d'un composé dihydrotriazine de formule 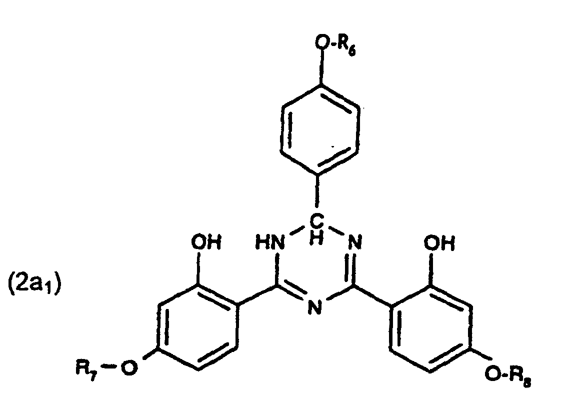 ou 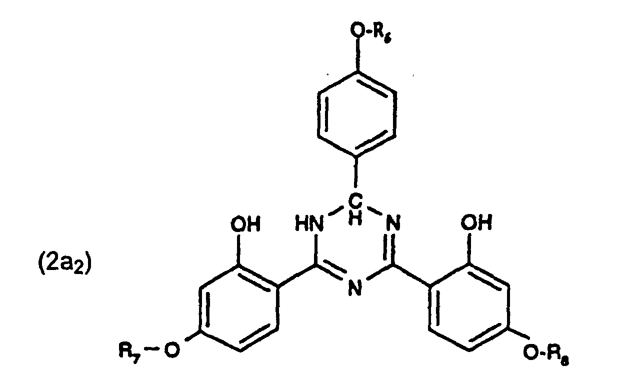 dans lesquelles
R₆, R₇ et R₈ ont les significations indiquées à la formule (1).

5. Utilisation non thérapeutique des bis-résorcinyltriazines de formules (2a) et (2b) selon la revendication 1 pour la protection de la peau et de la chevelure humaine et animale contre l'action nuisible du rayonnement UV.

6. Préparation cosmétique, contenant au moins un ou plusieurs composés des formules (2a) et (2b) selon la revendication 5 avec des supports ou adjuvants cosmétiquement acceptables.

7. Préparation selon la revendication 6, **caractérisée en ce qu'**elle contient d'autres substances protectrices anti-UV.

8. Préparation selon la revendication 6 ou 7, **caractérisée en ce qu'**elle contient comme autres substances de protection anti-UV des triazines, des oxanilides, des triazoles, des amides contenant des groupes vinyle ou des amides d'acide cinnamique.
